# EUROPEAN PATENT APPLICATION

(11) **EP 1 961 311 A1**
(43) Date of publication of application: **27.08.2008**
(21) Application number: 07102973.0
(22) Date of filing: 23.02.2007
(51) Int. Cl.: A23L 1/30, A61P 1/00, A61P 9/00, A61K 31/232

(54) **Food comprising polyunsaturated fatty acids for the prevention of chronic diseases**

(71) Applicant: Belovo S.A., Egg Science & Technology, 6600 Bastogne (BE)
(72) Inventor: De Meester, Fabien, B-6900, MARCHE (BE)
(74) Representative: pronovem

(57) **Abstract**

The present invention is related to a method for the prevention and possibly the treatment of chronic diseases, preferably chronic diseases selected from the group consisting of obesity, diabetes mellitus, cardiovascular diseases, hypertension, stroke and some types of cancers which are multigenic and multifactorial diseases that may affect a animal including man, by the administration of a sufficient amount of a (functional) food or feed to the diet of this animal.

## Description

### Field of the invention

The present invention is related to a method for the prevention and possibly the treatment of chronic diseases, preferably chronic diseases selected from the group consisting of obesity, diabetes mellitus, cardio- and cerebro-vascular diseases, auto-immune diseases, brain diseases, allergies, and some types of cancers which are multigenic and multifactorial diseases that may affect an animal including man, by the administration of a sufficient amount of a (functional) food or feed to the diet of this animal.

### Background of the invention

### PUFAs and cholesterol in land-based herbivorous animals

Cholesterol in the animal kingdom has been submitted to an evolutionary selective process, yet it sits and functions in animal cells for hundreds of million years. In the 6-million-year-old hominid species, cholesterol accounts for 70 g per 70 kg body weight (0.1 % w:w) and is distributed over all organs and tissues through blood circulation.

Cholesterol has been evolutionary selected as a unique blood and tissue active component in animals evolving in a wild environment. In such a land-based environment, body fats in herbivorous animals are characterized by a - 1:1 ratio between the two plant essential fatty acids (EFAs), linoleic acid (LA, C18:2ω6) and alpha-linolenic acid (ALA, C18:3ω3).

When this ratio of body fats is applied to modern livestock (including poultry), it appears that the blood (including yolk) fats of such animals are characterized by a 1:3 (~ 20% of variance) ratio of omega-6 to omega-3 total highly unsaturated fatty acids (HUFAs).

Human epidemiological studies tend to show that such 1:3 (~ 20% of variance) blood ratio of omega-6 to omega-3 total highly unsaturated fatty acids (HUFAs) prevents development (reduces the risk of development to ~ 0) of chronic diseases in man.

### Summary of the invention

The present invention is related to the use of a sufficient amount of a (functional) food or feed composition for the manufacture of a medicament to be administrated to an animal for the treatment and / or the prevention of a chronic disease affecting this animal, by a reduction of the blood omega-6 to omega-3 PUFAs ratio presented in said animal towards 1:1 (~ 50% of variance), through approaching a body fat essential fatty acids (EFAs) and / or a blood (or yolk) highly unsaturated fatty acids (HUFAs) ω6:ω3 ratios in said animal of 1:1 and / or 1:3 (~ 50% of variance), respectively.

Another aspect of the present invention is related to a method of prevention and possibly treatment of a animal that may suffer from a chronic disease, by the administration to said animal of a sufficient amount of a (functional) food or feed composition to reach a blood omega-6 to omega-3 PUFAs ratio in said animal of 1:1 (~ 50% of variance), through a reduction of body fat essential fatty acids (EFAs) and / or blood (or yolk) highly unsaturated fatty acids (HUFAs) ω6:ω3 ratios in said animal towards 1:1 and / or 1:3 (~50% of variance), respectively.

In the present invention, the essential fatty acid (EFAs) are linolenic acids (LA, C18:2ω6) and alpha-linoleic acid (ALA, C18 2.3 ω3), the highly unsaturated fatty acids (HUFAs) are the C20 and C22 fatty acids, among which arachidonic acid (AA, C20:4ω6), eicosapentaenoic acid (EPA, C20:5ω3), docosapentaenoic acid (DPA, C22:5ω3) and docosahexaenoic acid (DHA, C22:6ω3), and the polyunsaturated fatty acids (PUFAs) are the sum of the two (EFAs + HUFAs).

According to the invention, the chronic disease affecting the animal is selected from the group consisting of obesity, diabetes mellitus, cardio- and cerebro-vascular diseases, auto-immune diseases, brain diseases, allergies.

The (functional) food or feed composition is preferably selected from the group consisting of egg, meat, fat, fish, vegetables, seeds, oil, milk, bread or a mixture thereof that may possibly further processed and/or comprise additional carbohydrates, amino acids, anti-oxidants, vitamins and minerals.

### Detailed description of the invention

The inventors have discovered that when applied to modern livestock (poultry), a balanced ratio of essential fatty acids (ω6:ω3 EFAs = 1:1) in the animal (bird) body fat translates unexpectedly into a - 1:3 ratio of ω6 to ω3 in its blood (and yolk of the obtained egg) highly unsaturated fatty acids (ω6:ω3 HUFAs = 1:3).

It appears that Nature has selected cholesterol as an ideal tissue active component for land-based herbivorous animals complying with the following rules in terms of body fat and blood (yolk) fatty acid distribution (see table 1). Concurrently, such body fat and blood (yolk) fatty acid composition, when associated with the right diet-derived balance of essential amino acids, antioxidant vitamins and minerals, must be ideal for circulating lipoproteins (blood cholesterol) and tissue health & homeostasis, in land-based herbivorous animal species.

### CHD-Mortality and HUFAs in Man: Epidemiological Studies

Human prospective epidemiological evidence tends to show that the proportion of ω6 in blood total HUFAs is a potentially accurate parameter to estimate the risk of developing chronic diseases (Non-communicable diseases or NCDs), including obesity, diabetes mellitus, cardiovascular disease (CVD), hypertension, stroke, and some type of cancers, that are multigenic and multifactorial), such as exemplified by WEM Lands for coronary heart disease (CHD), and that a proportion of 25% ω6 in blood total HUFAs (ω6:ω3 HUFAs = 1:3) is an ideal epidemiologically-derived diet-related make-up in terms of protection against onset and development of such chronic diseases.

It is remarkable that the epidemiologically-determined safest blood HUFAs composition in man (25% ω6 HUFAs or ω6:ω3 HUFAs = 1:3) is consistent with that naturally established in wild land-based herbivorous animals, and that the latter naturally establishes itself in modern livestock raised on a wild-type plan diet.

### From Nutrition Facts to Nurturing Facts

Epidemiological evidence tends to support the view that Classic Nutrition Facts could be advantageously substituted for Modern Nurturing Facts to show essential rather than the non-essential fat content of the food (see table 2). Saturated and mono-unsaturated fats as well as cholesterol are non-essential for man. Saturated fats and cholesterol are secondary risk factors for chronic human diseases, whereas essential and highly unsaturated fatty acids determine body tissue composition, health and homeostasis.

The contribution of balanced Egg such as described in the European Patent EP1282367B1 to blood total ω6 HUFAs is calculated from an empirical equation derived by WEM Lands that is available on the US NIH website: http://efaeducation.nih.gov/sig/dietbalance.html

The mathematical model can be used to accurately test a diet or to approximately test a specific food item for its potential contribution to HUFA-related tissue inflammatory status and, in turn, to evaluate the potential risk of developing a chronic disease by keeping on such a diet for a long time or by eating such food item on a regular basis (Table 3).

This interactive learning website can also be used to help determine a potentially ideal dietary essential fatty acid distribution that is needed to maintain tissue health and homeostasis. In particular, linolenic acid (LA, C18:2w6) must be taken into consideration here since saturation of fatty acid physiological pathways leading to endogenous synthesis of eicosanoids (autacoids) in rat and man appears to require less than 1.0% of D.E.I. (22.22 Cal or 2.5 gm) as LA. Intakes in excess of that threshold seem to lead to impairment of ω3-HUFAs synthesis and accretion in tissues and organs. Computational analysis of US NIH website equation leads to an optimized dietary ratio of plant fats, ie ω6:ω3-EFAs = 1:3 (25% ω6-EFA/75% ω3-EFA) which maintains a proportion of 25% ω6 in blood total HUFAs. Interestingly, this distribution of essential fats is typical of ubiquitous greens and ancient seeds, such as flax, chia and perilla, on which man and wild-land based herbivorous animals have evolved.

### From Nutrition Facts to Nurturing Facts

ω6-rich grain-extracted table oils were not part of the food chain until Modern Agriculture came into play. From the classification obtained in table 3, it appears that most table oils seem to contribute to tissue inflammation and the onset of chronic diseases, so they could not have supported man's inception. Only certain Olive Oil (low in linolenic acid), Columbus Oil (described in the Patent Application W02005/020698 and which is a corrected olive oil), chia, flax and perilla oils tends to a composition that is compatible with man's tissue homeostasis. Modern meat obtained from livestock fed ω6-rich grains is also inflammatory, because of its high content of animal-derived ω6-HUFAs.

Among suitable functional food or feed composition to be used in the present invention, the person skilled in the art may select ocean fatty fishes or food or feed composition comprising them that are balancing foods in a modern type diet. Thanks to their high content in ω3-HUFAs, they can help in reducing the pro-inflammatory properties of modern oils & fats, and meats.

Very important is the fact that dairy products - including full fat milk, butter and cream - are low in essential fatty acids and do not contribute to HUFA-related tissue inflammation, yet they may contribute to increases in the classic TC:HDL and LDL:HDL ratios because of their saturated fatty acids content. However, the person skilled in the art may also obtain wild-type milk that present substantially lowered ratios of medium to long chain fatty acids (MCFA: -25%; LCFA: +25%). Therefore, wild-type dairies all belong to a healthy balanced diet where total fat and energy are kept under control.

Last but not least, the person skilled in the art should also take into consideration published and current recommendations for the use of fatty acid composition ((a) Simopoulos AP et al., Essentially of and recommended dietary intakes for ω-6 and ω-3 fatty acids. Food Rev International 2000; 16:113-117; (b) Scientific Committee on Food. Report on the Revision of Essential Requirements of Infant Formulae and Follow-on Formulae. SCF/CS/NUT/IF/65 Final (18 May 2003), EU Commission, Health & Consumer Protection Directorate General).

Genetically speaking, human beings have not changed over the 10,000 years since the development of modern agriculture. Healthy human beings exhibit balanced body fats (LA:ALA ratio) and / or a low proportion of ω6 in blood total HUFAs (ω6:ω3-EFAs = 1:1 and/or %ω6 in HUFAs = 25%). These compositions can be obtained and maintained through selection of land-based wild-type diets, complying with the same ratios when from animal origin (game, river fish) and in favour of omega-3 fatty acids (ω6:ω3-EFAs = 1:3) when from plant origin (leafy vegetables, some seeds & nuts).

Isolated transgenic fat-1 animal cells ((a) Kang ZB et al., Proc Natl Acad Sci USA 2001; 98:4050-4054; (b) Kang JX et al., Nature 2004;427:504) that are genetically given the ability to self balance ω6 and ω3 essential fatty acids confirm the wild hypothesis (ie ω6:ω3 PUFAs = ~1:1).

These Fat-1 transgenic mice were genetically engineered to express a fat-1 gene from the roundworm C. elegans and so engineered became capable of converting ω6 to ω3-PUFAs. These animals have a balanced ω6:ω3 fatty acid ratio in their tissues and organs, independent of diet, and are a unique animal model to validate the importance of tissue ω6- and ω3-PUFA proportions in man.

As expected, transgenic fat-1 mice appear to be more resistant against inflammatory agents and to recover faster in case of suffering from such induced degenerative diseases. (Hudert C et al., Proc Natl Acad Sci USA 2006; 103:11276-11281).

**Table 1**

| **Fatty acid (%)** | **Hen's body fat** | **Yolk total lipids** |
|---|---|---|
| C16:0 | 12.90 | 19.34 |
| C18:0 | 5.43 | 9.18 |
| C16:1ω7 | 2.34 | 3.17 |
| C18:1ω9 | 33.92 | 37.74 |
| C18:2ω6 | 21.16 | 13.59 |
| C18:3ω3 | 21.16 | 11.69 |
| C20:4ω6 | 0.04 | 0.81 |
| C20:5ω3 | 0.03 | 0.28 |
| C22:5ω3 | 0.02 | 0.43 |
| C22:6ω3 | 0.04 | 1.86 |
| ω6:ω3 EFAs | ~1:1 | - |
| ω6:ω3 HUFAs | - | ~1:3 |
| ω6:ω3 PUFAs | ~1:1 | ~1:1 |

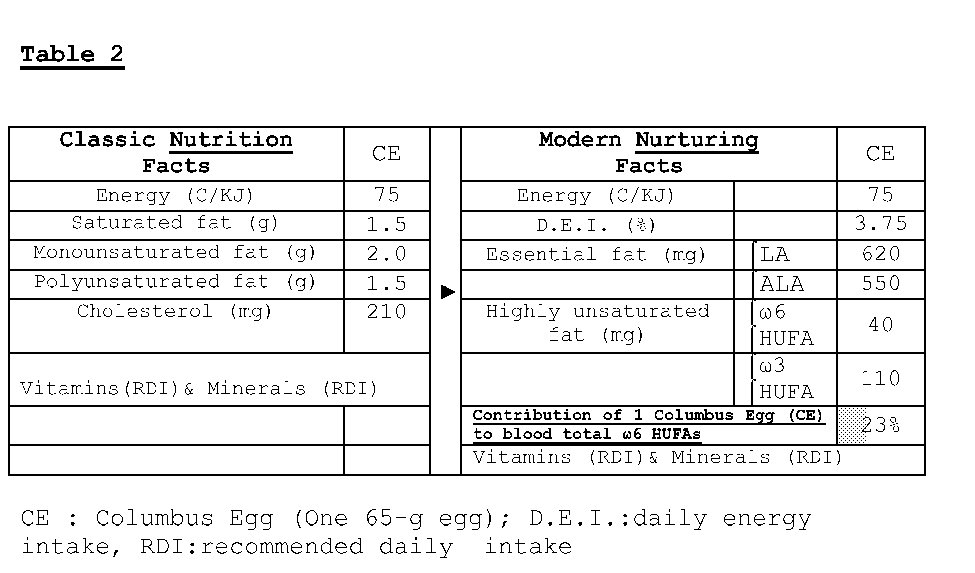

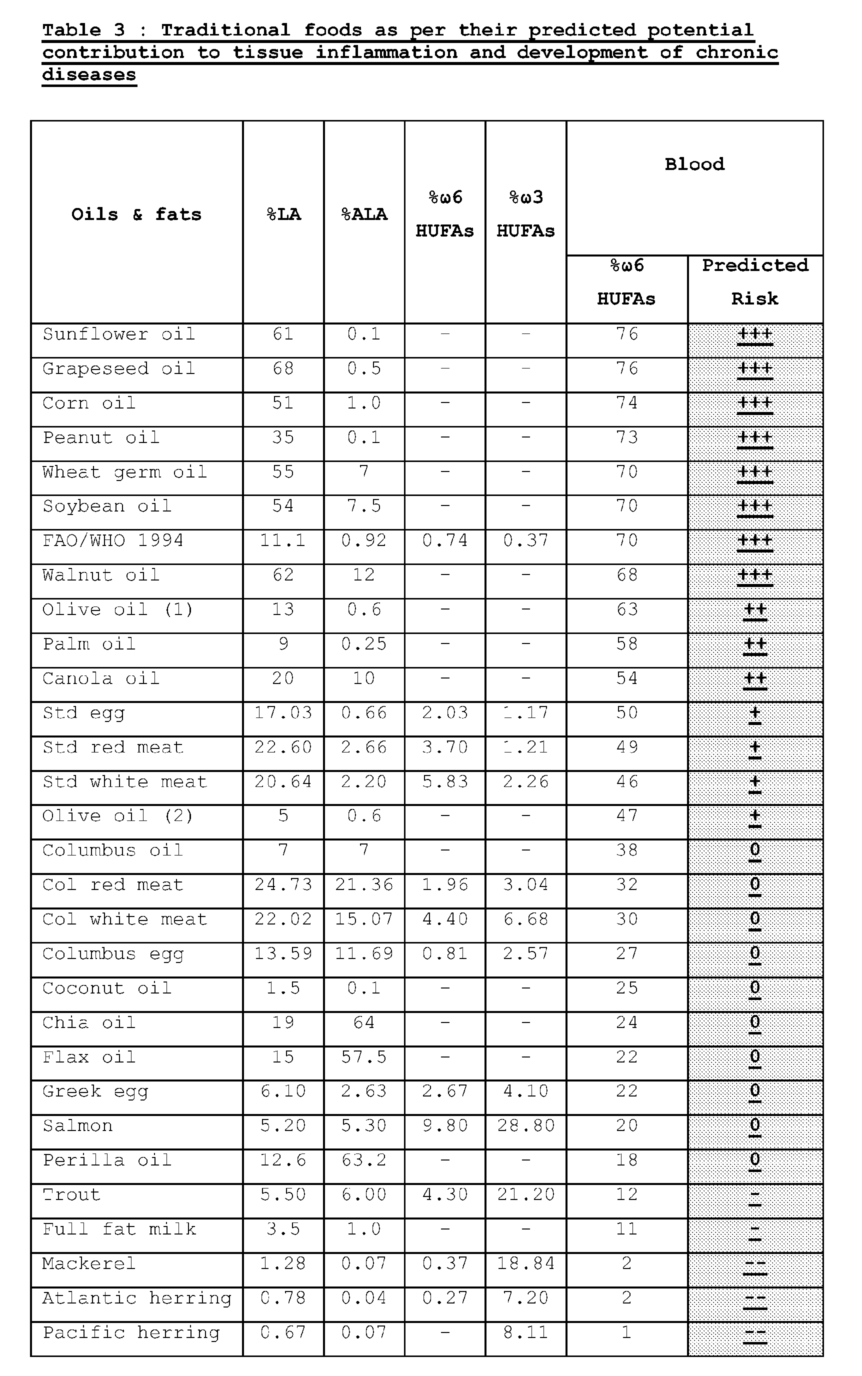

"Predicted HUFA-related Risk" (correlated here to the contribution of the food to %ω6 in blood total HUFAs) is calculated from US NIH website http://efaeducation.nih.gov/sig/dietbalance1/html for an intake of 20 g edible oil, 100-g edible egg (8.4 g total fatty acids), meat & fish (4.75 g total fatty acids), and milk (3.8 g total fatty acids) when contributing to a 30% fat-containing diet (2,222 Cal). Fatty acid compositional data are from The Lipid Handbook (Gunstone FD, Harwood JL & Padley FB, 2nd Ed), Chapman & Hall, 1994, ISBN 0 412 43320 6. Data on Chia oil are retrieved from www.eatchia.com Greek Ampelistra egg is from Simopoulos AP & Salem N Jr. N-3 fatty acids in eggs from range-fed Greek chickens. N Engl J Med 1989; 321:1412 (letter). FAO/WHO 1994 were recommended standards for infant formula; here computing was made for < 1 yr of age infant fed 900 ml milk formula daily. "Col" stands here for Columbus.

## Claims

1. The use of a sufficient amount of a (functional) food or feed composition comprising a ratio of ω6:ω3 polyunsaturated fatty acids (PUFAs) = 1:1 (-10%)for the manufacture of a medicament to be administrated to an animal, including man, for the treatment and/or the prevention of a chronic disease affecting said animal, by a reduction of body fat essential fatty acids (EFAs) and / or blood (or yolk) highly unsaturated fatty acids (HUFAs) ω6:ω3 ratios in said animal towards 1:1 and 1:3 - 10%, respectively.

2. The use according to the claim 1 wherein the essential fatty acids EFAs are linoleic acids and alpha-linolenic acids, the highly unsaturated fatty acids (HUFAs) are the C20 and C22 fatty acids, and the polyunsaturated fatty acids (PUFAs) are the sum of the two (EFAs + HUFAs).

3. The use according to the claim 1 or 2 wherein the chronic disease is selected from the group consisting of obesity, diabetes melitus, cardiovascular diseases, hypertension, stroke and cancer.

4. The use according to the claims 1 to 3 wherein the animal is a domestic animal.

5. The use according to the claim 1 to 3 wherein the animal is a man.

6. The use according to any of the preceding claims wherein the food or feed composition is selected from the group consisting of egg, meat, fat, fish, oil, vegetables, seeds, milk, bread or a mixture thereof and that may be possibly further processed and / or comprise essential aminoacids, antioxydants, vitamins and minerals.
